(19) Europäisches Patentamt
European Patent Office
Office européen des brevets

(11) EP 3 632 418 A1

(12) **EUROPEAN PATENT APPLICATION**
published in accordance with Art. 153(4) EPC

(43) Date of publication:
**08.04.2020 Bulletin 2020/15**

(21) Application number: **18809884.2**

(22) Date of filing: **31.05.2018**

(51) Int Cl.:
***A61K 31/047*** (2006.01) ***A23L 33/105*** (2016.01)
***A61K 36/185*** (2006.01) ***A61P 25/28*** (2006.01)

(86) International application number:
**PCT/JP2018/020944**

(87) International publication number:
**WO 2018/221650 (06.12.2018 Gazette 2018/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.06.2017 JP 2017109046**

(71) Applicant: **Santen Pharmaceutical Co., Ltd.**
**Higashiyodogawa-ku**
**Osaka-shi**
**Osaka 533-8651 (JP)**

(72) Inventors:
- **ITO, Takeshi**
  **Osaka-shi**
  **Osaka 530-8552 (JP)**
- **MAEDA, Hideto**
  **Tokyo 103-0022 (JP)**
- **HAYASHI, Sachiko**
  **Tokyo 103-0022 (JP)**
- **SAWANO, Yuri**
  **Tokyo 103-0022 (JP)**
- **NANRI, Tomoaki**
  **Tokyo 103-0022 (JP)**

(74) Representative: **Müller-Boré & Partner**
**Patentanwälte PartG mbB**
**Friedenheimer Brücke 21**
**80639 München (DE)**

(54) **BRAIN-DYSFUNCTION PREVENTING AND/OR IMPROVING COMPOSITION CONTAINING LUTEIN OR SALT THEREOF AND PROCESSED PRODUCT OF PLANT BELONGING TO GENUST**

(57) The present invention provides a novel composition for preventing and/or improving brain dysfunction comprising a lutein or a salt thereof and a plant product of the genus Trapa as active ingredients, particularly a composition for use as a pharmaceutical product and a food and beverage.

[FIG.1]

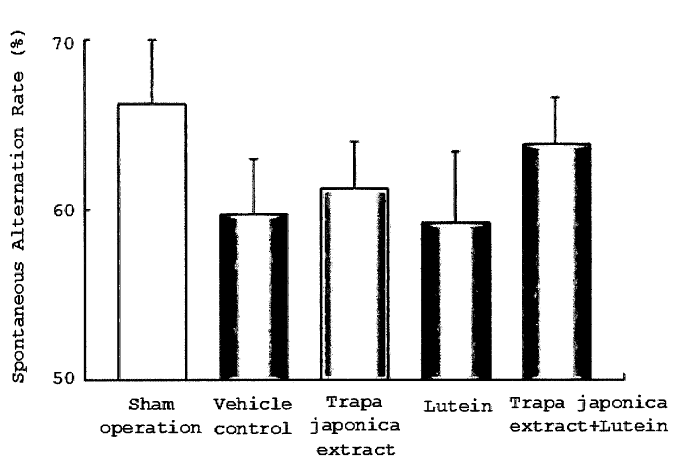




Printed by Jouve, 75001 PARIS (FR)

## Description

TECHNICAL FIELD

The present invention relates to a composition for preventing and/or improving brain dysfunction comprising a lutein or a salt thereof and a plant product of the genus Trapa as active ingredients.

BACKGROUND ART

Recently, the increase in patients suffering from brain diseases caused by reduced brain function due to aging such as memory impairment, dementia, and Alzheimer's disease has become a major social problem on the aging society. Also, it has beer, known that besides to aging, the accumulation of β-amyloid protein in the brain kills brain neurons and causes brain dysfunction such as dementia and Alzheimer's disease.

An agent for treating brain dysfunction such as dementia is limited to only a compound that crosses the blood-brain barrier, and thus there are few effective therapeutic agents. In addition, an agent for treating brain dysfunction is required to continue long-term administration, and thus produces strong side effects. Also, there is any problem when the agent is combined with other agent. As a result, it cannot be said that methods useful for the prophylaxis and/or treatment of diseases such as reduced brain function and brain disease have been established. Hence, the development of a food and beverage for preventing and improving brain dysfunction in the form that can be taken over a long term without side effect and toxicity, for example, in the form of supplement, besides a pharmaceutical product for preventing and treating brain dysfunction has been desired.

Lutein, which is one of carotenoids, has been known to be useful for keeping eye health as well as treating or preventing cataract and age-related macular degeneration. Also, lutein has been widely used as a pharmaceutical product or supplement in the field of ophthalmology. Lutein has anti-oxidant effect, and thus can be used for the age-related condition caused by the oxidative stress due to reactive oxygen, besides ophthalmic diseases. For example, Non-Patent Document 1 suggests that lutein may be useful for the treatment of age-related dementia and Alzheimer's disease.

The nut of a plant of the genus Trapa (Trapa) has nutritional fortification and promotes digestion. The effects for preventing hangover and improving visual loss are produced by boiling the whole plant and drinking the extract of the boiled plant. As a result, the plant of the genus Trapa is used as a medicine and a food.

Patent Document 1 discloses that the accumulation of advanced glycation end products (AGEs) produced by Maillard reaction is significantly increased in age-related diseases, that a plant extract of the genus Trapa produces Maillard reaction inhibitory effect and thus may be useful for the inhibition of symptoms associated with cataract, the decrease in elasticity of cartilage, and skin aging (e.g., the decrease in elasticity of cartilage, the formation of collagen cross-links that causes wrinkles and sagging, and pigmentation that causes dull skin), and that the extract may be useful for the prophylaxis and treatment of diabetes complications that have been known to involve Maillard reaction. Patent Document 2 discloses that a plant of the genus Trapa is useful for the improvement of infertility, that the inhibition of AGEs production is included as one of the factors related to the improvement of infertility, and that it is suggested that the AGEs involve in neurodegenerative disease such as Alzheimer's disease and Parkinson's disease, brain disorder caused by alcoholism, aging, and others.

On the other hand, it has not been specifically reported that a plant of the genus Trapa, for example, a plant product of the genus Trapa such as Trapa japonica extract is useful for the prophylaxis and improvement of brain dysfunction such as Alzheimer's disease and dementia. In addition, it has been unknown that the plant of the genus Trapa can improve reduced brain function associated with the accumulation of β-amyloid in the brain.

In addition, it has not been reported that a combination of a lutein and a plant product of the genus Trapa such as Trapa japonica extract was used for the prophylaxis and improvement of brain dysfunction.

PRIOR ART DOCUMENTS

PATENT DOCUMENTS

Patent Document 1: JP 2010-077123
Patent Document 2: JP 2016-145182

NON-PATENT DOCUMENTS

Non-Patent Document 1: James M. Stringham et al., "Nitric Oxide and Lutein: Function, Performance, and Protection of Neural Tissue", Foods 2015, 4, 678-389; doi: 10.3390/foods4040678

SUMMARY OF INVENTION

(PROBLEM TO BE SOLVED BY THE INVENTION)

An object of the present invention is to provide a composition for preventing and/or improving brain dysfunction comprising a lutein or a salt thereof and a plant product of the genus Trapa as active ingredients, particularly a composition for use as a pharmaceutical product and a food and beverage.

(MEANS FOR SOLVING THE PROBLEMS)

The present inventors have extensively studied to develop an effective treatment by a combination of ingredients derived from multiple natural products for brain dysfunction, particularly brain dysfunction associated with the accumulation of β-amyloid in the brain, and then have found that in the mice given the single-dose administration of β-amyloid, brain dysfunction is markedly improved when administering a lutein or a salt thereof in combination with a plant product of the genus Trapa or administering the ingredients as a combination product. Based upon the new findings, the present invention has been completed. Particularly, the present inventors have found that the combination administration of the active ingredients can markedly enhance the prophylaxis or improvement of brain dysfunction as compared to conventional lutein administration.

Specifically, the present invention provides the following embodiments.

[1] A composition for preventing and/or improving brain dysfunction comprising a lutein or a salt thereof and a plant product of the genus Trapa as active ingredients.
[2] The composition of the item [1], wherein the lutein is lutein, zeaxanthin, or meso-zeaxanthin.
[3] The composition of the item [1] or [2], wherein the plant product of the genus Trapa is at least one selected from the group consisting of Trapa acornis Nakano, Trapa incisa Sieb. et Zuc., Trapa japonica Flerov, Trapa bispinosa Roxb., Trapa natans L., and Trapa natans L.var.japonica Nakai.
[4] The composition of any of the items [1]-[3], wherein the plant product of the genus Trapa is a powdered product and/or an extract of pericarp of the plant of the genus Trapa.
[5] A composition for preventing and/or improving brain dysfunction comprising lutein or a salt thereof and a product of Trapa bispinosa Roxb. as active ingredients.
[6] The composition of the item [5], wherein the product of Trapa bispinosa Roxb. is a powdered product and/or an extract of pericarp of Trapa bispinosa Roxb.
[7] The composition of the item [5], wherein the product of Trapa bispinosa Roxb. is a plant extract of the genus Trapa.
[8] The composition of any of the items [1]-[7] for oral or parenteral administration.
[9] The composition of any of the items [1]-[8] which is a pharmaceutical composition.
[10] The composition of any of the items [1]-[8] which is a food composition.
[11] The composition of the item [10] which is health food, functional food, nutritional supplementary food, supplement, food with health claims, food for specified health uses, food with nutrient function claims, or food with function claims.
[12] The composition of any of the items [1]-[11] in the form of soft capsule, hard capsule, solution, jelly, gummy, tablet, or powder.
[13] The composition of any of the items [1]-[12], wherein the brain dysfunction is reduced brain function and a symptom associated thereto.
[14] The composition of the item [13], wherein the reduced brain function and the symptom associated thereto is the reduction in cognitive function, learning function, and memory function.
[15] The pharmaceutical composition of any of the items [1]-[9] and [12], wherein the brain dysfunction is a disease associated with accumulation of β-amyloid in the brain.
[16] The pharmaceutical composition of any of the items [1]-[9] and [12], wherein the brain dysfunction is a disease associated with the decrease in pyramidal cells in the brain.
[17] The pharmaceutical composition of the item [15] or [16], wherein the disease is Alzheimer's disease or dementia.
[18] A method of treating brain dysfunction, which comprises administering a therapeutically effective amount of a combination of a lutein or a salt thereof and a plant product of the genus Trapa to a patient in need thereof.
[19] The method of the item [18], wherein the lutein or the salt thereof is administered in combination with the plant product of the genus Trapa.

[20] The method of the item [19], wherein the lutein or the salt thereof and the plant product of the genus Trapa are administered simultaneously or separately with time-interval.
[21] A combination of a lutein or a salt thereof and a plant product of the genus Trapa for use in the prophylaxis and/or improvement of brain dysfunction.
[22] Use of a combination of a lutein or a salt thereof and a plant product of the genus Trapa in the manufacture of a medicament for preventing and/or treating brain dysfunction.
[23] A composition for preventing and/or improving brain dysfunction comprising a lutein or a salt thereof, wherein the lutein or the salt thereof is administered in combination with a plant product of the genus Trapa.
[24] A composition for preventing and/or improving brain dysfunction comprising a plant product of the genus Trapa, wherein the plant product of the genus Trapa is administered in combination with a lutein or a salt thereof.
[25] A method of enhancing the inhibitory effect of the accumulation of β-amyloid in brain of a composition comprising a lutein or a salt thereof by adding a plant product of the genus Trapa thereto.
[26] An agent for inhibiting the decrease in pyramidal cells in the brain comprising a lutein or a salt thereof and a plant product of the genus Trapa as active ingredients.
[27] A composition for preventing and/or improving brain dysfunction comprising a plant product of the genus Trapa as an active ingredient, wherein the brain dysfunction is a disease associated with the accumulation of β-amyloid in the brain.
[28] A method of treating brain dysfunction, which comprises administering a therapeutically effective amount of a plant product of the genus Trapa to a patient in need thereof, wherein the brain dysfunction is a disease associated with the accumulation of β-amyloid in the brain.
[29] A plant product of the genus Trapa for use in the prophylaxis and/or improvement of brain dysfunction, wherein the brain dysfunction is a disease associated with the accumulation of β-amyloid in the brain.
[30] Use of a plant product of the genus Trapa in the manufacture of a medicament for preventing and/or treating brain dysfunction, wherein the brain dysfunction is a disease associated with accumulation of β-amyloid in the brain.
[31] A composition for preventing and/or improving brain dysfunction comprising a plant product of the genus Trapa as an active ingredient, wherein the brain dysfunction is a disease associated with the decrease in pyramidal cells in the brain.
[32] A method of treating brain dysfunction, which comprises administering a therapeutically effective amount of a plant product of the genus Trapa to a patient in need thereof, wherein the brain dysfunction is a disease associated with the decrease in pyramidal cells in the brain.
[33] A plant product of the genus Trapa for use in the prophylaxis and/or improvement of brain dysfunction, wherein the brain dysfunction is a disease associated with the decrease in pyramidal cells in the brain.
[34] Use of a plant product of the genus Trapa in the manufacture of a medicament for preventing and/or treating brain dysfunction, wherein the brain dysfunction is a disease associated with the decrease in pyramidal cells in the brain.
[35] A composition for preventing and/or improving brain dysfunction comprising a lutein or a salt thereof as an active ingredient, wherein the brain dysfunction is a disease associated with the accumulation of β-amyloid in the brain.
[36] A method of treating brain dysfunction, which comprises administering a therapeutically effective amount of a lutein or a salt thereof to a patient in need thereof, wherein the brain dysfunction is a disease associated with the accumulation of β-amyloid in the brain.
[37] A lutein or a salt thereof for use in the prophylaxis and/or improvement of brain dysfunction and a plant product of the genus Trapa, wherein the brain dysfunction is a disease associated with the accumulation of β-amyloid in the brain.
[38] Use of a lutein or a salt thereof in the manufacture of a medicament for preventing and/or treating brain dysfunction, wherein the brain dysfunction is a disease associated with the accumulation of β-amyloid in the brain.
[39] A composition for preventing and/or improving brain dysfunction comprising a lutein or a salt thereof as an active ingredient, wherein the brain dysfunction is a disease associated with the decrease in pyramidal cells in the brain.
[40] A method of treating brain dysfunction, which comprises administering a therapeutically effective amount of a lutein or a salt thereof to a patient in need thereof, wherein the brain dysfunction is the disease associated with a decrease in pyramidal cells in the brain.
[41] A lutein or a salt thereof for use in the prophylaxis and/or improvement of brain dysfunction and a plant product of the genus Trapa, wherein the brain dysfunction is a disease associated with the decrease in pyramidal cells in the brain.
[42] Use of a lutein or a salt thereof in the manufacture of a medicament for preventing and/or treating brain dysfunction, wherein the brain dysfunction is a disease associated with a decrease in pyramidal cells in the brain.

(Effects of the Invention)

According to the present invention, the administration of a lutein or a salt thereof in combination with a plant product of the genus Trapa or the administration of the ingredients as a combination product is expected to improve reduced brain function to normal state. Also, the lutein or a salt thereof and the plant product of the genus Trapa are expected to be used as a pharmaceutical composition and a food composition.

Brief Description of the Drawings

Fig. 1 shows the results of Y-maze test in each group (sham operation, control, Trapa japonica extract, Lutein, Trapa japonica extract + Lutein). The bar graphs in Fig. 1 show the spontaneous alternation rate (%) in each group.

Fig. 2 shows the area of pyramidal cell layer of the hippocampus CA1 region ($\mu m^2$) in Klüver-Barrera (KB) stained tissue specimen in each group (sham operation, control, Trapa japonica extract, Lutein, Trapa japonica extract + Lutein).

Hereinafter, the present invention is explained in detail.

As used herein, "a lutein" refers to lutein or a metabolite thereof derived from a natural product such as plant, animal, and microorganism, for example, lutein or a metabolite thereof extracted or purified from a plant such as marigold, spinach and kale, egg yolk, retina, yellow spot, and others or chemically-synthesized lutein or a metabolite thereof produced by general synthesis method. Also, the types, the production areas, and the production process of the raw material of lutein or a metabolite thereof derived from natural products are not particularly limited.

In addition, the lutein may be used in various physiologically or pharmaceutically acceptable salt forms. The salt also includes an ester. Examples of the salt include a salt with an inorganic acid such as hydrochloric acid, hydrobromic acid, hydroiodic acid, nitric acid, sulfuric acid, and phosphoric acid; a salt with an organic acid such as acetic acid, fumaric acid, maleic acid, succinic acid, citric acid, tartaric acid, adipic acid, gluconic acid, glucoheptonic acid, glucuronic acid, terephthalic acid, methanesulfonic acid, lactic acid, hippuric acid, 1,2-ethanedisulfonic acid, isethionic acid, lactobionic acid, oleic acid, pamoic acid, polygalacturonic acid, stearic acid, tannic acid, trifluoromethanesulfonic acid, benzenesulfonic acid, p-toluenesulfonic acid, lauryl sulfate, methyl sulfate, naphthalenesulfonic acid, and sulfosalicylic acid; a salt with halogen ion such as bromide ion, chloride ion, and iodide ion; a salt with alkali metal such as lithium, sodium, and potassium; a salt with alkali earth metal such as calcium and magnesium; a salt with metal such as iron and zinc; a salt with ammonia; and a salt with organic amine such as triethylenediamine, 2-aminoethanol, 2,2-iminobis(ethanol), 1-deoxy-1-(methylamino)-2-D-sorbitol, 2-amino-2-(hydroxymethyl)-1,3-propanediol, procaine, and N,N-bis(phenylmethyl)-1,2-ethanediamine. Also, the ester may be in the form of monoester or diester, and examples thereof include an ester with acid such as acetic acid, lauric acid, myristic acid, caprylic acid, pentadecanoic acid, palmitic acid, palmitoleic acid, heptadecanoic acid, elaidic acid, ricinoleic acid, petroselinic acid, vaccenic acid, eleostearic acid, punicic acid, licanic acid, parinaric acid, gadoleic acid, 5-eicosenoic acid, 5-docosenoic acid, cetolic acid, ercinoic acid, 5,13-docosadienoic acid, selacholic acid, decenoic acid, stering acid, dodecenoic acid, oleic acid, stearic acid, eicosapentaenoic acid, docosahexaenoic acid, linoleic acid, linolenic acid, and arachidonic acid.

Examples of the lutein include lutein, zeaxanthin, and meso-zexanthin, but are not limited thereto. The lutein or a salt thereof of the present invention is preferably lutein or a salt thereof. The lutein or a salt thereof of the present invention may be used two or more.

As used herein, "lutein" is a compound represented by chemical name: (3R,3'R,6'R)-β,ε-carotene-3,3'-diol, and also the following formula:

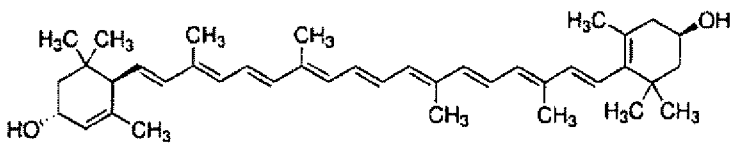

Lutein is one of carotenoids enriched in green and yellow vegetables, and is also one of main carotenoids in the body.

As used herein, "zeaxanthin" is a compound represented by chemical name: (3R,3'R)-β,β-carotene-3,3'-diol, and also the following formula:

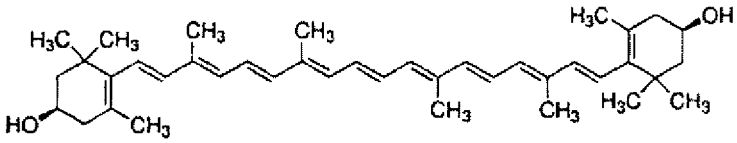

Zeaxanthin is lipophilic compound similar to β-carotene, and the structural isomer of lutein (lutein metabolite). Zeaxanthin is enriched in the yellow spot and has the effect for protecting the yellow spot.

As used herein, "meso-zeaxanthin" is a compound represented by chemical name: (3R,3'S)-β,β-carotene-3,3'-diol, and also the following formula:

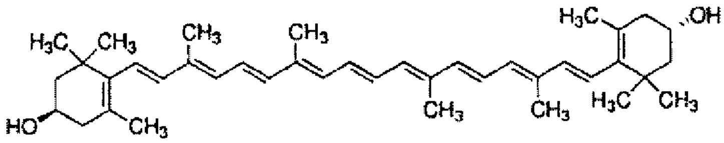

Meso-zeaxanthin is the stereoisomer of zeaxanthin (lutein metabolite). Meso-zeaxanthin is supposed to have similar effects to zeaxanthin.

The lutein or a salt thereof may be added in an amount of, for example, 0.1-90% by weight, preferably 0.5-80% by weight, more preferably 1-70% by weight, furthermore preferably 1.5-60% by weight, particularly preferably 2-50% by weight, relative to the total weight of the composition of the present invention.

The lutein or a salt thereof may be administered in an amount of, for example, 0.1-500 mg, preferably 0.5-250 mg, more preferably 1-100 mg, furthermore preferably 3-75 mg, particularly preferably 5-50 mg per day for adults.

As used herein, "plant of the genus Trapa" refers to annual waterweed of Trapaceae. Specific examples of the plant of the genus Trapa include Trapa acornis Nakano, Trapa incisa Sieb. et Zuc., Trapa japonica Flerov, Trapa bispinosa Roxb., Trapa natans L., Trapa natans L.var.japonica Nakai, but are not limited thereto. The plant of the genus Trapa of the present invention is preferably Trapa japonica Flerov, Trapa bispinosa Roxb. and Trapa natans L.var.japonica Nakai, and particularly preferably Trapa bispinosa Roxb.

As used herein, "plant product of the genus Trapa" refers to a powder and an extract prepared or manufactured from the whole or each part of said plant of the genus Trapa, and a dry product thereof. Examples of the parts of the plant of the genus Trapa include flower, spike, pericarp, nut, sarcocarp, stem, leaf, branch, branches and leaves, trunk, bark, rhizome, root bark, root, seed, gall, heartwood, aerial part, and underground part. The parts thereof are particularly preferably pericarp. The plant product of the genus Trapa can be prepared or manufactured by, for example, crushing each part as it is or the part cut to an appropriate size in fine powder, squeezing the part or extracting the part with solvent, and optionally drying the resulting product. Also, two or more parts of the plant of the genus Trapa can be appropriately mixed to prepare or manufacture the plant product.

Examples of the extraction methods of the plant of the genus Trapa include batch extraction and continuous extraction by column extraction, but are not limited thereto. As extraction solvents, water, an organic solvent or a mixture thereof may be used. Examples of the organic solvent include lower alcohol such as methanol and ethanol, chloroform, ethyl acetate and n-hexane. The extraction solvent is preferably water, methanol or ethanol. Also, two or more of the solvents may be used.

The amount of the extracted solvent used can appropriately be determined depending on the type or the part of the plant of the genus Trapa and the type of extraction solvent. The weight ratio of the plant of the genus Trapa and the extraction solvent is, for example, 1:2 to 1:30, preferably 1:3 to 1:20, particularly preferably 1:5 to 1:10.

Also, the extraction time is, for example, few minutes to 30 days, preferably 1 hour to 15 days. The extraction temperature is, for example, 5 to 100°C.

The plant extract of the genus Trapa may use the extracted solution itself. If necessary, the solution may be applied to purification treatment. The purification treatment can be performed according to conventional methods. For example, the plant extract of the genus Trapa can be purified through conventional filtration methods. The resulting filtrate can then be treated by any operations such as vacuum concentration and lyophilization to obtain the dried plant product of the genus Trapa.

Examples of the plant product of the genus Trapa may include an extract of pericarp of the plant or a dry product thereof. The extract of pericarp of the plant of the genus Trapa or a dry product thereof may be prepared or manufactured by, for example, extracting pericarp of the plant of the genus Trapa with water or a lower alcohol such as methanol and ethanol, filtrating and concentrating the extract, and optionally drying it with an addition of an additive such as an excipient.

The plant product of the genus Trapa is particularly preferably Trapa japonica extract which is the product of Trapa bispinosa Roxb. The Trapa japonica extract is hot water extract made from pericarp of Trapa bispinosa Roxb.,

and contains a large amount of polyphenols which are anti-glycation active ingredients.

The plant product of the genus Trapa may be added in an amount of, for example, 0.1-99% by weight, preferably 1-95% by weight, more preferably 10-90% by weight, furthermore preferably 25-85% by weight, and particularly preferably 60-80% by weight, relative the total weight of the composition of the present invention.

The plant product of the genus Trapa may be administered in an amount of, for example, 0.1-2000 mg, preferably 1-1500 mg, more preferably 10-1000 mg, furthermore preferably 25-500 mg, particularly preferably 50-300 mg per day for adults.

The plant product of the genus Trapa includes polyphenol. In the present invention, the lower limit of the amount of polyphenol relative to the total weight of the plant product of the genus Trapa is, for example, 0.1% by weight, preferably 5% by weight, and particularly preferably 10% by weight. Also, the upper limit thereof is, for example, 80% by weight, preferably 70% by weight, and particularly preferably 60% by weight. In addition, the upper and lower limits may be used in combination. More specifically, the amount of polyphenol relative to the total weight of the plant product of the genus Trapa is, for example, 0.1-80% by weight, preferably 5-70% by weight, and particularly preferably 10-60% by weight. Also, the amount of polyphenol can be measured by any method such as FOLIN-CIOCALTEU method.

When the plant product of the genus Trapa is an extract of pericarp of a plant of the genus Trapa, the lower limit of the amount of polyphenol relative to the total weight of the extract is, for example, 0.1% by weight, preferably 1% by weight, more preferably 5% by weight, furthermore preferably 10% by weight, and particularly preferably 20% by weight. Also, the upper limit thereof is, for example, 90% by weight, preferably 80% by weight, more preferably 70% by weight, furthermore preferably 65% by weight, and particularly preferably 60% by weight. In addition, the upper and lower limits may be used in combination. More specifically, the amount of polyphenol relative to the total weight of the extract is, for example, 0.1-90% by weight, preferably 1-80% by weight, more preferably 5-70% by weight, furthermore preferably 10-65% by weight, and particularly preferably 20-60% by weight.

As used herein, "brain dysfunction" refers to disorder that causes reduced brain function related to brain itself and nervous system, for example, disorder with one or more symptoms such as aphasia, attention disorder, memory impairment, behavioral and emotional disorder, hemispatial neglect, executive function disorder, apraxia, hemiasomatognosia, topographical disorientation, and agnosia.

As used herein, "reduced brain function" refers to reduced brain function caused by aging or disease, and also includes the maintenance of brain function. In addition, the reduced brain function includes reduced brain function associated with the accumulation of $\beta$-amyloid in the brain and the decrease in pyramidal cells in the brain. Examples of the disease that causes reduced brain function include aging, Alzheimer's disease, and dementia. Also, "symptom associated thereto" used herein means symptom caused by reduced brain function, and examples thereof include the reduction in cognition, learning function, memory, intelligence, and motivation.

As used herein, "composition for preventing and/or improving reduced brain function" refers to a composition for the prophylaxis, improvement and/or treatment of a disease that causes reduced brain function and a symptom associated thereto. As used herein, "improvement" means changing symptom and condition of a disease for the better, the prevention or delay of the exacerbation of symptom and condition of a disease, the reversion, prevention or delay of the progression of symptom of a disease, or the treatment of a disease, and "prophylaxis" means the prevention or delay of the onset of a disease in an applicable subject or the reduction in the risk of the onset of a disease in an applicable subject.

As used herein, "treatment" refers to any treatment (e.g. improvement, alleviation, and prevention of the progression) of diseases.

As used herein, "patient" refers to human and an animal such as dog, cat, house. The patient is preferably a mammal, and more preferably human.

As used herein, "therapeutically effective amount" refers to any amount of an active ingredient that will elicit the biological or medical response of a tissue, system, animal or human that is being sought by a researcher or clinician. Also, when the "therapeutically effective amount" is administered to a mammal in order to treat a disease, it refers to the amount of an active ingredient (e.g., a lutein or a salt thereof, a plant product of the genus Trapa) which is sufficient to produce such therapeutic effect on the disease. The "therapeutically effective amount" will vary depending on various factors such as an active ingredient, a disease and severity thereof, as well as the age and body weight of mammal to be treated.

In addition, the "effective amount" refers to an amount of an active ingredient (e.g., a lutein, a plant product of the genus Trapa) that produces an acute or chronic therapeutic effect upon administration in an appropriate dose. The therapeutic effect includes the prevention, correction, inhibition, or reversal of the symptoms, signs and underlying pathology of disease/condition and related complications to any detectable extent.

Such effective amount includes the amount of the lutein or the plant product of the genus Trapa of the present invention alone, the amount of the combination of the lutein or the plant product of the genus Trapa of the present invention, and/or the amount of the active ingredient of the present invention used in combination with other active ingredient.

As used herein, "pyramidal cell" refers to projective excitatory neuron which is the principal neuron of the cerebral cortex. Pyramidal cells play an important role in the signal transduction within and between the areas of the cerebral cortex as well as the signal transduction from the cortex to the subcortex. The decrease in the number of pyramidal cells due to the cell atrophy or damage causes brain dysfunction such as dementia and Alzheimer's disease.

The combination of the lutein or a salt thereof and the plant product of the genus Trapa is expected to prevent and/or improve brain dysfunction such as Alzheimer's disease and dementia to inhibit the decrease in pyramidal cells in the brain.

The combination ratio of the lutein or a salt thereof and the plant product of the genus Trapa of the present invention may be determined so that effects for preventing and/or improving brain dysfunction can be produced, but is not particularly limited. The combination ratio may be selected from, for example, 1:100 to 100:1, preferably 1:50 to 5:1, and furthermore preferably 1:20 to 2:1, but is not limited thereto.

In the combination of the lutein or a salt thereof and the plant product of the genus Trapa of the present invention, both active ingredients may be administered separately or together in a single preparation. In addition, one ingredient in the combination of the present invention may be administered before, simultaneously with, or after the administration of the other ingredient in the combination. These ingredients may be formulated into a single dosage form or two separate ones.

Examples of the combination form of the lutein or a salt thereof and the plant product of the genus Trapa of the present invention include, but are not limited to, the following forms (I) and (II):

(I) the form of a single preparation (one composition, combination product) containing both the lutein or a salt thereof, and the plant product of the genus Trapa; and

(II) the form comprising a preparation (composition) containing the lutein or a salt thereof, and a preparation (composition) containing the plant product of the genus Trapaas separate preparations.

In such form (II), the preparations may be administered simultaneously or separately at different points in time with an appropriate interval, and an appropriate dosing regimen may be employed so that desired effects for preventing and/or improving brain dysfunction can be produced.

When the active ingredients are formulated into a single dosage form, the plant product of the genus Trapa is mixed in a ratio of 0.01-100 parts by weight, preferably 0.2-50 parts by weight, more preferably 0.5-20 parts by weight, per 1 part by weight of the lutein or a salt thereof, but is not limited to thereto. Also, the single dosage form includes the sum of the active ingredients in 0.1-99% by weight in the composition of the dosage form, but is not limited thereto.

The composition of the present invention may add other active ingredient (e.g., compound for producing an auxiliary effect) and an additive according to well-known methods as long as they prevent the effect for preventing and/or improving brain dysfunction.

Examples of the other active ingredient, which may be added in the composition of the present invention, include vitamin A group; carotenoids (excluding xanthophyll); vitamin B group; vitamin C group; vitamin D group; vitamin E group; tocotrienols; glutathione and its derivative or a salt thereof; polyphenols such as catechin, anthocyanin, tannin, rutin, isoflavone, chlorogenic acid, ellagic acid, curcumin, coumarin; linoleic acid, $\alpha$- or $\gamma$-linolenic acid, arachidonic acid, eicosapentaenoic acid, clupanodonic acid, docosahexaenoic acid and their derivative or a salt thereof; a protein selected from collagen, elastin, fibronectin, or keratin, a derivative and a hydrolyzate thereof; $\alpha$-hydroxy acid such as glycolic acid, lactic acid, malic acid, citric acid, salicylic acid and their derivative or a salt thereof; a natural product and an extract thereof such as deproteinized serum, spleen, placenta, cockscomb, royal jelly, yeast, lactic acid bacterium, bifidobacteria, Ganoderma lingzhi, ginseng root, swertia herb, rosemary, Phellodendron bark, Allium sativum, hinokitiol, cepharanthine, aloe, salvia, arnica, camomile, Betula platyphylla, Hypericum erectum, Eucalyptus, SapindaceaeSapindus mukorossi, Inula japonica, Spatholobi Caulis, Cassia nomame Honda, Mulberry bark, Angelica sinensis, Bistorta officinalis subsp. japonica, Sophora angustifolia, hawthorn, Lilium candidum, hop, Rosa multiflora, Coix seed, Houttuynia cordata, seaweed, fermented soybeans, Cymbopogon citratus, Hibiscus, Ginkgo biloba leaf extract, phosphatidylserine; an adenylic acid derivative such as adenosine triphosphate, adenosine diphosphate, and adenosine monophosphate; minerals such as iron, vanadium, molybdenum, manganese, copper, potassium, zinc, selenium, and iodine; monosaccharides such as mannitol, xylitol, and glucosamine; polysaccharides such as hyaluronic acid, chondroitin sulfate, dermatan sulfate, heparan sulfate, heparin, keratan sulfate, glycogen, chitin, and chitosan; nucleic acids such as deoxyribonucleic acid and ribonucleic acid; other glycyrrhizic acid, guanine, mucin, ubiquinone, $\alpha$-lipoic acid, octacosanol, allysine, alliin, and a mixture thereof. The other active ingredient is preferably vitamin A group; carotenoids (excluding xanthophyll); vitamin B group; vitamin C group; vitamin D group; vitamin E group; tocotrienols; glutathione and its derivative or a salt thereof; polyphenols such as catechin, anthocyanin, and ellagic acid; linoleic acid, $\alpha$- or $\gamma$-linolenic acid, arachidonic acid, eicosapentaenoic acid, clupanodonic acid, docosahexaenoic acid and their derivative or a salt thereof; a protein selected from collagen, elastin, fibronectin, or keratin, a derivative and a hydrolyzate thereof; minerals such as copper and zinc; mucin, ubiquinone, $\alpha$-lipoic acid, and a mixture thereof. The other active ingredient is furthermore preferably vitamin A

group; carotenoids (excluding xanthophyll); vitamin C group; vitamin E group; tocotrienols; glutathione and its derivative or a salt thereof; polyphenols such as catechin, anthocyanin, and ellagic acid; eicosapentaenoic acid, docosahexaenoic acid and their derivative or a salt thereof; minerals such as copper and zinc; mucin, ubiquinone, α-lipoic acid, and a mixture thereof.

The other active ingredient may be added in an amount of, for example, 0.1-99% by weight, preferably 1-90% by weight, more preferably 5-80% by weight, furthermore preferably 10-70% by weight, and particularly 20-60% by weight. Also, two or more of the other active ingredients may be combined.

Examples of the additive, which may be added in the composition of the present invention, include an excipient, a thickener, and an emulsifier (e.g., beeswax, glycerin fatty acid ester).

The composition of the present invention may be administered orally or parenterally (e.g., subcutaneously, intramuscularly or intravenously). The composition of the present invention may be mixed with an additive such as an excipient, a thickener, and an emulsifier to prepare in a desired dosage form. Examples of the dosage form of the composition of the present invention include a supplement form such as soft capsule, hard capsule, solution, jelly, gummy, tablet, powder and gel, but are not limited thereto.

The composition of the present invention may be used as a food and beverage or a pharmaceutical product (including a pharmaceutical product for mammal other than human) . In the present invention, the amount of the composition of the present invention to be added for a food and beverage or a pharmaceutical product may be determined depending on various factors, but are not limited to, the applicable purpose (e.g., target disease, type of symptom), the applicable object (e.g., human, animals such as human (preferably mammal, particularly preferably dog, cat)), the part of the applicable object, the sex and age of the applicable object, the form of a food and beverage or a pharmaceutical product, the administration or ingestion method and the frequency thereof, and the preference.

In the present invention, when the composition of the present invention is prepared as a pharmaceutical product (such as medicine, quasi-drug), the lutein or a salt thereof and the plant product of the genus Trapa may be mixed with other active ingredient, a pharmaceutically acceptable carrier, and an additive. For example, a pharmaceutically acceptable or an additive such as a binder, a disintegrant, a lubricant, a wetting agent, a buffer, a preservative, and a flavor may be added to formulate into a desired dosage form. Examples of the pharmaceutical dosage form include the forms of injection preparation, external preparation, inhalant, suppository, film agent, troche, solution, powder, tablet, granule, capsule, syrup, eye drop, eye wash, and nasal drop, but are not limited thereto. Among them, the dosage form is preferably the dosage form suitable for oral administration (that is, oral medicine), and particularly preferably the dosage form such as troche, solution, powder, tablet, granule, capsule, soft capsule, and syrup. The dosage form can be used as, for example, a medicine for preventing and/or treating reduced brain function.

In the present invention, when the composition of the present invention is prepared as a food composition (e.g., health food, functional food, nutritional supplementary food, supplement, food with health claims, food for specified health uses, food with nutrient function claims, a food with function claims), the lutein or a salt thereof and the plant product of the genus Trapa may be mixed with a sweetener, a food coloring, a preservative, a thickener, a stabilizer, a gelling agent, a pasting agent, an antioxidant, a color former, a bleaching agent, a fungicide (an antimold agent), a yeast food, a gum base, a flavor, an acidifier, a seasoning, an emulsifier, a pH adjuster, salt water, a leavening agent, an enrichment, or other food and beverage material to formulate into a desired form. Examples of the form of the composition of the present invention include the forms of supplement-type food such as gelling agent, granule, fine granule, capsule, soft capsule, tablet, powder, solution, and semi-solid agent; beverage such as carbonated beverage, refreshing beverage, milk beverage, alcoholic beverage, fruit juice beverage, tea, nutrient beverage; powdered beverage such as powdered juice, powdered soup; confectionery such as gum, tablet, candy, cookie, gummi, rice cracker, biscuit, jelly; bread, noodles, cereal, jam, and seasoning, but are not limited thereto. The forms may be used as, for example, a food and beverage for preventing and/or improving brain dysfunction. Also, the forms may be used as nutraceuticals such as nutritional supplementary food, food with function claims, food for specified health uses, and food for patients besides general food and beverage.

When the composition of the present invention is used as a food composition, the indication "prophylaxis (or inhibition) and/or improvement (or treatment) of reduced brain function" may be used in the composition. Also, the indication "prophylaxis (or inhibition) and/or improvement (or treatment) of reduced brain function" may be indicated as, for example, "the composition improves reduced brain activity caused by aging of healthy middle-aged and older people and enhances the accuracy of memory (memorizing and remembering words, numbers, figures) which is part of cognitive functions and the accuracy of judgment", "the composition enhances the accuracy of memory (remembering perceived or recognized matters) which is part of cognitive functions", "function to maintain memory (ability to remember words, images of objects, and location information) which is part of cognitive functions", "the composition has function to support memory of information such as numbers, words, figures, and situations which is part of cognitive functions", "the composition has function to maintain memory (ability to memorize and remember actions and judgments that occur in daily life) which is part of cognitive functions", "the composition enhances the accuracy of memory (memorizing and remembering words, numbers, figures) which is part of cognitive functions and the accuracy of judgment", "the composition

enhances the accuracy of memory (remembering perceived or recognized matters) which is part of cognitive functions", "the composition enhances the accuracy of memory (remembering recognized words, images of objects, and experiences) which is part of cognitive functions and the accuracy of judgment", "the composition maintains memory (words and person's faces) which is part of cognitive functions", "the composition maintains memory (ability to memorize and remember words, figures, and others) which is part of cognitive functions", "the composition maintains memory (ability to remember matters seen and heard a while ago) which is part of cognitive functions", "the composition has function to maintain memory (ability to memorize and remember information seen and heard in daily life) which is one of cognitive functions", "the composition has function to maintain memory (ability to memorize and remember information such as numbers, words, and situations) which is part of cognitive functions", "the composition has function to maintain memory (ability to memorize and remember seen and heard contents) which is part of cognitive functions", "the composition has function to support memory (ability to memorize and remember the information seen and heard in daily life, images of words and objects, and location information) which is part of cognitive functions", "the composition has function to maintain memory (ability to memorize and remember information seen and heard in daily life), "function to support memory of information such as numbers, words, figures, stories, colors, and situations (ability to remember matters and keep them in memory) which is part of cognitive functions", "the composition has useful function to reduce fatigue in persons who feel tired in daily life, to alleviate the feeling due to fatigue, for example, the feeling that I cannot concentrate and I have reduced attention, and to improve the efficiency of works that require simple memory and judgment", "the composition maintains memory, attention, judgment, and spatial ability, which are part of cognitive functions, reduced with aging of middle-aged and older people", "the composition maintains memory (ability to memorize and remember words, figures, and others), which is part of cognitive functions, of middle-aged and older people", "effect for maintaining part of cognitive functions reduced with aging of middle-aged and older people (memory, attention, judgment, and spatial ability)", "the composition has function to support memory of information such as numbers, words, figures, and situations, which is part of cognitive functions, of middle-aged and older people", "the composition maintains memory (ability to memorize and remember words, figures, and others), which is part of cognitive functions, reduced with aging of healthy middle-aged and older people", "the composition has function to support memory of information such as numbers, characters, figures, spaces, which is part of cognitive functions, reduced with aging of middle-aged and older people", "the composition has function suitable for persons who are concerned about memory reduced with age (is useful for maintaining, retrieving, and restoring memory)", "the composition plays a role in alleviating memory function (function to memorize information as well as remember and judge it smoothly), which is part of cognitive functions, reduced with age", "the composition maintains memory (ability to remember matters seen and heard a while ago), which is part of cognitive functions, of brain", "the composition has function to maintain motor function which is part of cognitive functions", "the composition improves visual function (linkage and accommodation of function entered from eyes such as vision, eye movement, and linkage and accommodation functions of both eyes and the treatment function to perceive, memorize, and imagine visual information in the brain) reduced with reduced brain function", "the composition plays a role in improving the visual function, which is part of cognitive functions, reduced with age", "the composition maintains visual function, which is part of cognitive functions, in the brain", "the composition has function to maintain visual function which is part of cognitive functions", and also may be indicated as indications similar thereto. The indication may be conveyed in a direct or indirect manner. Examples of the direct indication include description on product itself, description on packaging such as package, container, label and tag, and examples of the indirect indication include advertisement and promotional activity such as transaction document, manual, catalog, website, storefront, exhibition, signboard, bulletin board, newspaper, magazine, TV, radio, and e-mail.

## EXAMPLES

Hereinafter, the present invention is illustrated in more detail in Formulation Examples and the results of Test Examples. The present invention, however, is not intended to be limited to them by any means.

### Formulation Examples

Typical formulation examples of the present invention are shown below. The amount of each ingredient formulated in the following formulation examples is the content in 1 capsule when it is used as soft capsule.

### Formulation Example 1

| In 1 soft capsule | |
|---|---|
| Lutein | 7.5 mg |
| Trapa japonica Extract | 33 mg |
| Glycerin fatty acid ester | Appropriate quantity |

Formulation Example 2

| In 1 soft capsule | |
|---|---|
| Lutein | 7.5 mg |
| Trapa japonica Extract | 33 mg |
| Vitamin E | 75 mg |
| Glycerin fatty acid ester | Trace quantity |

Formulation Example 3

| In 1 soft capsule | |
|---|---|
| Lutein | 7.5 mg |
| Trapa japonica Extract | 33 mg |
| Docosahexaenoic acid | 100 mg |
| Glycerin fatty acid ester | Appropriate quantity |

Formulation Example 4

| In 1 soft capsule | |
|---|---|
| Zeaxanthin | 7.5 mg |
| Trapa japonica Extract | 33 mg |
| Glycerin fatty acid ester | Appropriate quantity |

Formulation Example 5

| In 1 soft capsule | |
|---|---|
| Meso-zeaxanthin | 7.5 mg |
| Trapa japonica Extract | 33 mg |
| Glycerin fatty acid ester | Appropriate quantity |

Test Example 1

(Method)

Slc:ddY (SPF:Specific-Pathogen-Free) mice (Supply Resource: Japan SLC, Inc.) received the administration a sample (Vehicle control, Trapa japonica extract, Lutein, or Trapa japonica extract + Lutein) and then the injection of β-amyloid solution. For the mice, Y-maze test was performed to evaluate the improvement effects of Trapa japonica extract, Lutein, and a combination thereof on learning disorder. The test was performed twice. The Y-maze test was performed according to Takayoshi MAMIYA et al., "Effects of Pre-Germinated Brown Rice on β-Amyloid Protein-Induced Learning and Memory Deficits in Mice", Biol. Pharm. Bull., July 2004, 27(7), 1041-1045 to evaluate the effects.

[Animal used]

Male, 5-week-old, Slc:ddY (SPF) mouse(Body weight: 23~28 g: n=32)

According to the following procedure, model mice were made using the above mice: Each mouse was anesthetized with the intraperitoneal administration (dosing volume: 10 mL/kg) of pentobarbital sodium (TOKYO CHEMICAL INDUSTRY CO., LTD.) at 40 mg/kg. After anesthesia, levobupivacaine hydrochloride (POPSCAINE® 0.25% injection, Maruishi Pharmaceutical Co., Ltd.) (0.1 mL) was administered subcutaneously into the scalp of the mouse. The hair on the top of the mouse was trimmed, and the head part was fixed to a brain stereotactic device. The scalp of the mouse was sterilized with iodine tincture, and then the head part was incised to expose the skull and the connective tissue on the skull was removed with a cotton swab. The treated skull was dried with a blower to find the position of bregma more easily. A hole for inserting a stainless steel pipe was drilled in the skull at 1 mm to the right and 0.2 mm behind bregma with a dental drill. The stainless steel pipe connected to a silicon tube with an outside diameter of 0.5 mm and a micro syringe was perpendicularly inserted to a depth of 2.5 mm from the bone surface. 3 μL of a β-amyloid solution (6 μmol/3 μL) was injected (in the sham operation group, 3 μL of water for injection was injected) into the cerebral ventricle with a microsyringe pump for 3 minutes. After the injection, the inserted stainless steel pipe was allowed to stand for 3 minutes, and then the pipe was slowly removed. After removing the stainless steel pipe, the cranial was closed with a non-absorbable myelostatic agent (NESTOP®, Alfresa Pharma Co., Ltd.) and the scalp was sutured. The mouse were removed from the brain stereotactic device and returned to the breeding cage.

The β-amyloid solution was prepared by dissolving β-amyloid (Amyloid-β Protein(25-35); Polypeptide Laboratories) to 2 mM with water for injection and incubating the solution in the constant temperature storage at 37°C for 4 days. Water for injection was used as the vehicle for sham operation.

[Sample]

- Vehicle control (Water for injection + Safflower oil)

- Trapa japonica extract

To the raw material for Trapa japonica extract (Hayashikane Sangyo Co., Ltd.) was added water for injection (Otsuka Pharmaceutical Factory, Inc.) to prepare 0.2 mg/mL of Trapa japonica extract.

- Lutein

To lutein (Katra Phytochem [India] Private Limited) was added safflower oil (Wako Pure Chemical Industries, Ltd.) to prepare 0.04 mg/mL of Lutein.

[Administration of sample]

The Slc:ddY mice were randomly classified into 5 groups (Sham operation group, Vehicle control group, Trapa japonica extract group, Lutein group, Trapa japonica extract + Lutein group) so that the average weight of the mice in each group is almost equal (n=10 per each group). The administration of sample was started for each group at the same time. The administration of the β amyloid solution and the administration of the vehicle for sham operation (water for injection) were done on the 8 day from the start date of the sample administration. The type and the dosing of sample administered for each group are as follows. The mice received the gavage administration of each sample at 10 mL/kg once per day for 14 days. For the sample, water for injection or Trapa japonica extract was administered, and then safflower oil or lutein was administered 10 minutes after the first administration. Also, the test mice in all of the groups were freely given MF feed (manufactured by Oriental Yeast Co., Ltd.) besides each sample.

[Table 1]

| Group | Dosing mg/kg | Sample | n |
|---|---|---|---|
| 1. Sham operation | - | Water for Injection + Safflower Oil | 10 |
| 2. Vehicle control | - | Water for Injection + Safflower Oil | 10 |
| 3. Trapa japonica extract | 2 | Trapa Japonica Extract + Safflower Oil | 10 |
| 4. Lutein | 0.4 | Water for Injection + Lutein | 10 |
| 5. Trapa japonica extract + Lutein | 2+0.4 | Trapa Japonica Extract + Lutein | 10 |

[Y-maze test (Spontaneous Alternation Test)]

A plastic Y-shaped maze with 3 identical arms at an angle of 120° from each other, each arm having a length of 39.5 cm, a width of floor of 4.5 cm and a height of wall of 12 cm (UNICOM) was used as the experimental device for the Y-maze test.

Each mouse was placed in any one of the arms of Y-shaped maze about 1 hour after the administration on the 14th day and allowed to explore in the maze freely for 8 minutes. The order of the arms moved by the mouse at that time was recorded. The number of moves to another arm was defined as the total number of entries and a combination of three different arms selected consecutively among the total moves was defined as the number of spontaneous alternation to calculate spontaneous alternation rate (%) according to the following equation.

Spontaneous Alternation Rate (%) = [the number of spontaneous alternation / (the total number of entries - 2)] x 100

(Results)

The results for the Y-maze test in each group for the model mice are shown in Fig. 1 and Table 2. The total number of entries, the number of spontaneous alternation, and the number of spontaneous alternation in all groups were expressed as the average value of n= 10.

[Table 2]

| | The total number of entries (Count) | The number of spontaneous alternation (Count) | Spontaneous alternation rate (%) |
|---|---|---|---|
| Sham operation | 40.1 | 25.6 | 66.2 |
| standard error | 3.8 | 3.1 | 2.7 |
| Vehicle control | 36.3 | 20.5 | 59.8 |
| standard error | 3.2 | 1.9 | 1.6 |
| Trapa japonica extract | 36.7 | 21.3 | 61.3 |
| standard error | 2.7 | 2.0 | 3.0 |
| Lutein | 37.0 | 21.2 | 59.3 |
| standard error | 4.1 | 3.0 | 2.7 |
| Trapa japonica extract + Lutein | 36.0 | 22.2 | 63.9 |
| standard error | 2.7 | 2.6 | 3.6 |

It was found that the spontaneous alternation rate of the vehicle control group was significantly decreased as compared to that of the sham operation group ($p<0.05$ in the t-test). That is, the mice of the vehicle control group had the significantly-reduced ability to memorize the arm that has already moved in the Y maze as compared to those of the sham operation group. This suggests that the administration of amyloid $\beta$ caused memory impairment.

On the other hand, the spontaneous alternation rates of the Trapa japonica extract group and the Trapa japonica extract + Lutein group were increased as compared to that of the vehicle control group.

As a result, it was demonstrated that the mice of the Trapa japonica extract group and the Trapa japonica extract + Lutein group had the improved ability to memorize the arm that has already moved in the Y maze as compared to those of the vehicle control group. It was found that the spontaneous alternation rate of the Lutein group was not improved, but the administration of Trapa japonica extract and the combination administration of Trapa japonica extract + Lutein was effective for improving memory impairment.

According to the above test results, it was demonstrated that the administration of Trapa japonica extract or Trapa japonica extract + Lutein improved the reduced ability to memorize the arm that has already moved in the Y maze and greatly increased the spontaneous alternation rate. This showed that the administration of Trapa japonica extract

or Trapa japonica extract + Lutein could improve brain dysfunction and Alzheimer's dementia associated with β-amyloid protein. In particular, the administration of Trapa japonica extract + Lutein markedly improved brain dysfunction and Alzheimer's dementia associated with by β-amyloid protein.

Test Example 2

(Method)

The brain was isolated from the mice in each group of Test Example 1 to prepare histopathological specimens, and the histopathological assessment of the prepared histopathological specimens was performed. Such test was performed twice.

[Isolation of brain]

Each mouse in each group of Test Example 1 was anesthetized with an isoflurane solution and the chest of the mouse was opened to expose the heart. The exposed heart was cut into the auricle thereof. A polypropylene disposable syringe with an injection needle was used, the injection needle thereof was inserted from left ventricle and about 30 mL of ice-cooled saline was injected into the heart, and then the heart was perfused with about 30 mL of 10vol% formalin neutral buffer solution. After perfusion, the death of the mouse was observed to isolate the whole brain, and then the isolated brain was immersed in 10vol% formalin neutral buffer solution.

[Preparation of histopathological specimen]

The isolated brain was embedded in paraffin according to any conventional method, one histopathological section was discontinuously prepared at around bregma-1.70 mm and around bregma-2.80 mm, respectively, and then Klüver-Barrera stained (KB stained) tissue specimens were prepared from each section. The cut brain was stored in 10vol% formalin neutral buffer solution.

[Histopathological assessment]

1) For each specimen, the images of pyramidal cell layers of the hippocampus CA1 region closest to bregma-1.70 mm and bregma-2.80 mm from the optical microscope image (George Paxinos and Keith B.J. Franklin. (2013). The Mouse Brain in stereotaxic coordinates. Elsevier) were observed to select either of left or right brain as an object for assessment.
2) Images were taken with a 10x objective lens using a digital camera for microscopes.
3) The taken digital images were combined to make one image as the object for assessment.
4) The area of pyramidal cell layer of the hippocampus CA1 region was measured using a two-dimensional image analysis software WinROOF2015 system Version 3.6 (Mitani Corporation).

(Results)

The measurement results of the area of pyramidal cell layer of the hippocampus CA1 region are shown in Fig. 2 and Table 3. The results in all of the groups are represented as the average value of n=2.

[Table 3]

| | Area of pyramidal cell layer ($\mu m^2$) |
|---|---|
| Sham operation | 193755 |
| Vehicle control | 174729 |
| Trapa japonica extract | 202088 |
| Lutein | 224874 |
| Trapa japonica extract + Lutein | 239495 |

It was found that the area of pyramidal cell layer of the hippocampus CA1 region of the vehicle control group was decreased as compared to that of the sham operation group. As a result, it was shown that the administration of β-amyloid produced the decrease in pyramidal cells in the brain.

On the other hand, it was found that the area of pyramidal cell layer of the hippocampus CA1 region of the Trapa japonica extract group, the Lutein group, and the Trapa japonica extract + Lutein group was increased as compared to that of the vehicle control group.

According to the above test results, it was demonstrated that the administration of Trapa japonica extract, Lutein or Trapa japonica extract + Lutein inhibited the decrease in pyramidal cells in the brain. This shows that the administration of Trapa japonica extract, Lutein, or Trapa japonica extract + Lutein could prevent or improve brain dysfunction and Alzheimer's dementia associated with the decrease in pyramidal cells in the brain. In particular, the administration of Trapa japonica extract + Lutein markedly improved brain dysfunction and Alzheimer's dementia associated with the decrease in pyramidal cells in the brain.

Industrial Applicability

The present invention uses a lutein or a salt thereof and a plant product of the genus Trapa as active ingredients, and thus are useful as not only a pharmaceutical composition but also a food composition such as a food and beverage and a supplement. As a result, the present invention can provide a composition for preventing and/or improving brain dysfunction with high safety in a wide range of fields.

## Claims

**1.** A composition for preventing and/or improving brain dysfunction comprising a lutein or a salt thereof and a plant product of the genus Trapa as active ingredients.

**2.** The composition of claim 1, wherein the lutein is lutein, zeaxanthin, or meso-zeaxanthin.

**3.** The composition of claim 1 or 2, wherein the plant product of the genus Trapa is at least one selected from the group consisting of Trapa acornis Nakano, Trapa incisa Sieb. et Zuc., Trapa japonica Flerov, Trapa bispinosa Roxb., Trapa natans L., and Trapa natans L.var.japonica Nakai.

**4.** The composition of any one of claims 1-3, wherein the plant product of the genus Trapa is a powdered product and/or an extract of pericarp of the plant of the genus Trapa.

**5.** A composition for preventing and/or improving brain dysfunction comprising lutein or a salt thereof and a product of Trapa bispinosa Roxb. as active ingredients.

**6.** The composition of claim 5, wherein the product of Trapa bispinosa Roxb. is a powdered product and/or an extract of pericarp of Trapa bispinosa Roxb.

**7.** The composition of claim 5, wherein the product of Trapa bispinosa Roxb. is a plant extract of the genus Trapa.

**8.** The composition of any one of claims 1-7 for oral or parenteral administration.

**9.** The composition of any one of claims 1-8 which is a pharmaceutical composition.

**10.** The composition of any one of claims 1-8 which is a food composition.

**11.** The composition of claim 10 which is health food, functional food, nutritional supplementary food, supplement, food with health claims, food for specified health uses, food with nutrient function claims, or food with function claims.

**12.** The composition of any one of claims 1-11 in the form of soft capsule, hard capsule, solution, jelly, gummy, tablet, or powder.

**13.** The composition of any one of claims 1-12, wherein the brain dysfunction is reduced brain function and a symptom associated thereto.

14. The composition of claim 13, wherein the brain hypofunction and the symptom associated thereto is a decline in cognitive function, learning function, and memory function.

15. The composition of any one of claims 1-9 and 12, wherein the brain dysfunction is a disease associated with accumulation of β-amyloid in the brain.

16. The composition of any one of claims 1-9 and 12, wherein the brain dysfunction is a disease associated with the decrease in pyramidal cells in the brain.

17. The composition of claim 15 or 16, wherein the disease is Alzheimer's disease or dementia.

18. An agent for inhibiting the decrease in pyramidal cells in the brain comprising a lutein or a salt thereof and a plant product of the genus Trapa as active ingredients.

[FIG.1]

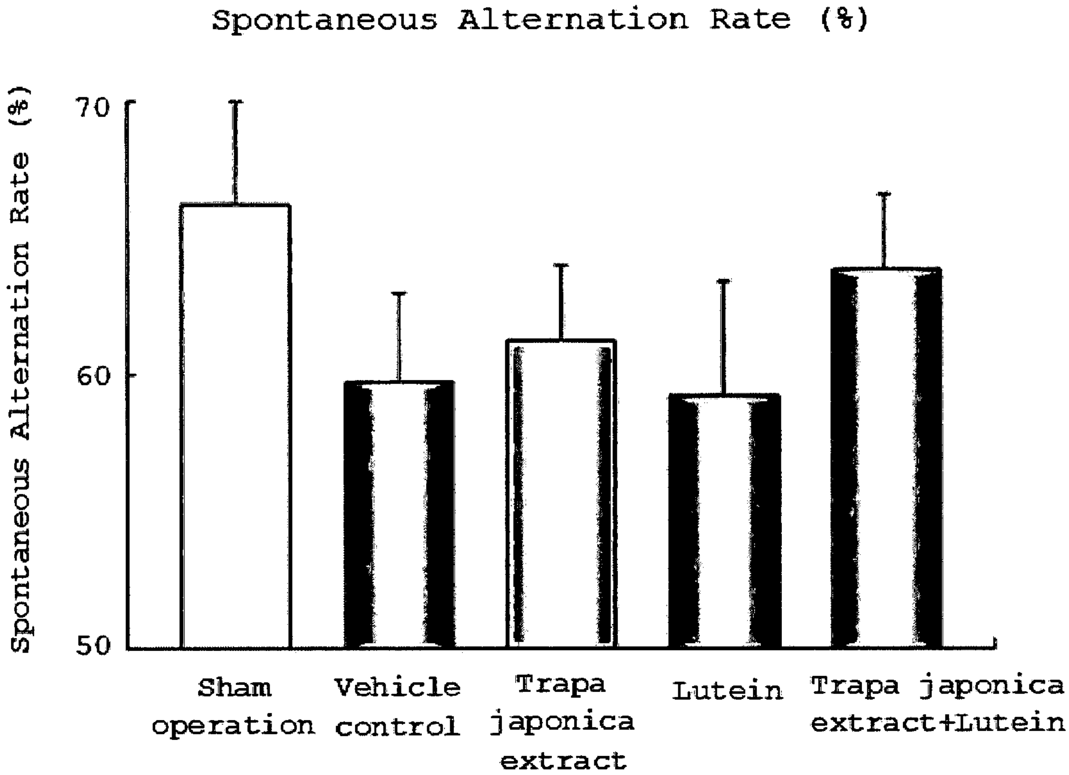
Spontaneous Alternation Rate (%)
Spontaneous Alternation Rate (%)
70
60
50
Sham operation
Vehicle control
Trapa japonica extract
Lutein
Trapa japonica extract+Lutein

[FIG.2]

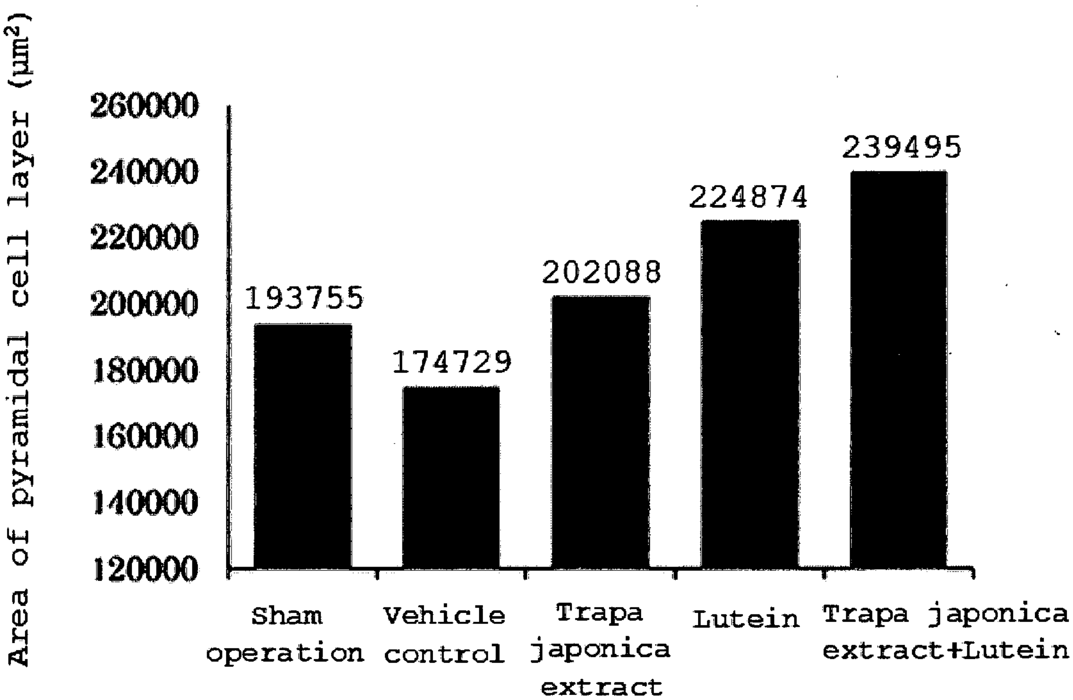
Area of pyramidal cell layer (μm²)
260000
240000
220000
200000
180000
160000
140000
120000
193755
174729
202088
224874
239495
Sham operation
Vehicle control
Trapa japonica extract
Lutein
Trapa japonica extract+Lutein

## INTERNATIONAL SEARCH REPORT

International application No. PCT/JP2018/020944

**A. CLASSIFICATION OF SUBJECT MATTER**

Int.Cl. A61K31/047(2006.01)i, A23L33/105(2016.01)i, A61K36/185(2006.01)i, A61P25/28(2006.01)i

According to International Patent Classification (IPC) or to both national classification and IPC

**B. FIELDS SEARCHED**

Minimum documentation searched (classification system followed by classification symbols)
Int.Cl. A61K31/047, A23L33/105, A61K36/185, A61P25/28

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
JSTPlus/JMEDPlus/JST7580 (JDreamIII), CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| Y<br>A | LAKEY, Beitia Johant et al., "Anti-amyloid aggregation activity of novel carotenoids:Implications for alzheimer's drug discovery", Clinical Interventions in Aging, 15 May 2017, vol. 12, pp. 815-822, ISSN 1176-9092, abstract, table 1 | 1-17<br>18 |
| Y<br>A | KATAYAMA, Shigeru et al., "Apricot Carotenoids Possess Potent Anti-amyloidogenic Activity in Vitro", J. Agric. Food Chem., 2011, vol. 59, no. 23, pp. 12691-12696, ISSN 0021-8561, abstract, fig. 4, 5 | 1-17<br>18 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 August 2018 (24.08.2018) | 04 September 2018 (04.09.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No. PCT/JP2018/020944

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | LIU, Tao. et al., "Lutein protects against β–amyloid peptide-induced oxidative stress in cerebrovascular endothelial cells through modulation of Nfr-2 and NF-κb", Cell Biology and Toxicology, 2016, vol. 33, no. 1, pp. 57-67, ISSN 0742-2091, abstract, fig. 1-4, scheme 1 | 1-17<br>18 |
| Y<br>A | NAKAGAWA, Kiyotaka et al., "Amyloid β–induced erythrocytic damage and its attenuation by carotenoids", FEBS Letters, 2011, vol. 585, no. 8, pp. 1249-1254, ISSN 0014-5793, abstract, fig. 4, 5 | 1-17<br>18 |
| Y<br>A | JP 2013-510129 A (DSM IP ASSETS B.V.) 21 March 2013, paragraph [0012], fig. 1-6 & US 2012/0232162 A1, paragraphs [0018]-[0023], fig. 1-6 & WO 2011/054944 A1 & EP 2498765 A1 & CN 102612364 A & MX 2012005441 A & KR 10-2012-0115253 A & CN 107050000 A | 1-17<br>18 |
| Y<br>A | JP 2016-520540 A (OMNIACTIVE HEALTH TECHNOLOGIES LIMITED) 14 July 2016, claims 2, 9, tables 3, 4, 6-8, paragraphs [0095]-[0097] & US 2016/0051490 A1, claims 15, 26, paragraphs [0074], [0084]-[0087], [0092]-[0094] & WO 2014/155189 A1 & EP 2978418 A1 & IN 1208MU2013 A & AU 2014242655 A & CA 2907886 A & CN 105120853 A & HK 1211836 A & MX 2015013624 A & SG 11201507973T A & KR 10-2015-0137088 A | 1-17<br>18 |
| Y<br>A | AMBIKAR, D. B. et al., "Neuroprotective effect of hydroalcoholic extract of dried fruits of Trapa bispinosa Roxb on lipofuscinogenesis and fluorescence product in brain of D-galactose induced ageing accelerated mice", Indian. J. Exp. Biol., 2010, vol. 48, no. 4, pp. 378-382, ISSN 0019-5189, abstract, table 1 | 1-17<br>18 |
| Y<br>A | AMBIKAR, D. B. et al., "Effect of hydroalcoholic extract of dried FRUITS of trapa natans Lon animal models of cognitive dysfunction", Indian Drugs, March 2017, vol. 54, no. 3, pp. 39-43, ISSN 0019-462X, (abstract), [online] STN, EMBASE, AN.20170372794, DN.616396782 | 1-17<br>18 |
| Y<br>A | VYAWAHARE, N. S. et al., "Evaluation of neurophamacological activity of hydroalcoholic extract of fruits of trapa bispinosa in laboratory animals", Int. J. Pharmacy. Pharm. Sci., 2010, vol. 2, no. SUPPL.2, pp. 32-35, ISSN 0975-1491, abstract, fig. 1 | 1-17<br>18 |

Form PCT/ISA/210 (continuation of second sheet) (January 2015)

INTERNATIONAL SEARCH REPORT

International application No. PCT/JP2018/020944

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | KR 2010-86680 A (KOREA INSTITUTE OF SCIENCE AND TECHOLOGY) 02 August 2010, paragraphs [0014], [0101], test example 2, tables 1-4 (Family: none) | 1-17<br>18 |
| A | YOKOYAMA, Koichi et al., "Protective effects of Choto-san and hooks and stems of uncaria sinensis against delayed neuronal death after transient forebrain ischemia in gerbil", Phytomedicine, 2004, vol. 11, no. 6, pp. 478-489, ISSN 0944-7113, abstract, fig. 3-6, tables 3, 4 | 1-18 |
| A | 新藤一敏他，菱の実及び菱茶に含有される抗酸化活性物質， 日本家政学会誌, 2013, vol. 64, no. 7, pp. 353-359, ISSN 0913-5227, abstract, fig. 1, table 2, (SHINDO, Kazutoshi et al., "Antioxidative Compounds Contained in the Seed with Hard Shell of Trapa Japonica Flerov. and its Herbal Tea", Journal of home economics of Japan) | 1-18 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2010077123 A
- JP 2016145182 A


### Non-patent literature cited in the description


- **JAMES M. STRINGHAM et al.** Nitric Oxide and Lutein: Function, Performance, and Protection of Neural Tissue. *Foods,* 2015, vol. 4, 678-389
- **TAKAYOSHI MAMIYA et al.** Effects of Pre-Germinated Brown Rice on β-Amyloid Protein-Induced Learning and Memory Deficits in Mice. *Biol. Pharm. Bull.,* July 2004, vol. 27 (7), 1041-1045
- **GEORGE PAXINOS ; KEITH B.J. FRANKLIN.** The Mouse Brain in stereotaxic coordinates. Elsevier, 2013